# EUROPEAN PATENT SPECIFICATION

(11) **EP 0 707 473 B1**
(45) Date of publication and mention of the grant of the patent: **25.03.1998**
(21) Application number: 94916352.1
(22) Date of filing: 02.06.1994
(51) Int. Cl.: A61K 9/16

(54) **ORAL PHARMACEUTICAL COMPOSITIONS COMPRISING A PROTEIN OR PEPTIDE, AN ANTIBODY AND POLYMERIC BEADS**
ORALE, PHARMAZEUTISCHE ZUSAMMENSETZUNGEN DAS EIN PROTEIN ODER PEPTIDE, EINE ANTIKOERPER UND POLYMERKUGELN ENTHALTEN
COMPOSITIONS PHARMACEUTIQUES POUR ADMINISTRATION PAR VOIE ORALE COMPRENANT UNE PROTEINE OU UN PEPTIDE, UN ANTICORPS ET DES PERLES POLYMERES

(30) Priority: 03.06.1993 GB 9311454
(43) Date of publication of application: 24.04.1996
(73) Proprietor: THE BABRAHAM INSTITUTE, Babraham, Cambridge CB2 4AT (GB)
(72) Inventor: SMITH, Michael, Waddington, London SW13 0DB (GB)
(74) Representative: Chapman, Paul William
(86) International application number: GB9401201
(87) International publication number: WO9428879

(56) References cited:
- EP-A- 0 238 396
- WO-A-92/17167

## Description

The present invention relates to improved pharmaceutical compositions and, in particular, to compositions for oral administration containing biologically active materials, particularly peptides or proteins.

Proteins and peptides are used in the treatment of very many diseases or conditions and protein hormones such as insulin, calcitonin and growth hormones and also proteins such as erythropoietin, plasminogen activators and their precursors, interferons, interleukins and blood clotting factors are merely some of the numerous examples and, in addition, many compounds which are used as vaccines are proteins or peptides.

However, although Proteins and peptides are so widely used in medicine, they must generally be administered by intravenous or intramuscular injection since, even though they can be enterically coated so as to survive the conditions of the stomach, they are not easily adsorbed through the gut wall. Injection is not a popular route of administration with patients and this often leads to poor patient compliance. In addition, with conditions such as diabetes where daily injections have to be given, problems often arise because of the necessity of injecting many times in the same area. In order to overcome these problems, it would be helpful to develop ways of administering these compounds *via* the oral route since this is the simplest and most popular route with patients.

There have been many attempts to enable proteins and peptides to be administered orally and these have included various formulations in which the protein or peptide is encapsulated in liposomes or formulations including penetration enhancers. However, liposomes are often unreliable and penetration enhancers may increase the uptake of unwanted substances as well as the substances on which they are intended to act.

WO92/17167 discloses complexes and compositions for oral delivery of a substance or substances to the circulation or lymphatic drainage system of a host. The complexes comprise a microparticle coupled to at least one carrier, the carrier being capable of enabling the complex to be transported to the circulation or lymphatic drainage system via the mucosal epithelium of the host, and the microparticle entrapping or encapsulating, or being capable of entrapping or encapsulating, the substance(s). Examples given of suitable carriers are mucosal binding proteins, bacterial adhesins, viral adhesins, toxin binding subunits, lectins, Vitamin B₁₂ and analogues or derivatives of Vitamin B₁₂ possessing binding activity to Castle's intrinsic factor.

WO-A-8705505 discloses oral compositions of insulin coated onto particles and covered with a lipid coat.

JP-A-55017328 discloses water-in-oil-in-water emulsions containing insulin and intended for oral administration.

However, the present inventor has adopted a completely different approach from any which has previously been used and has discovered that it is possible to make use of M cells as a route for peptide absorption from the gut lumen into the body.

M cells occur in the epithelial lining of the Peyer's patch lymphoid follicles of the intestine. They are epithelial cells which, probably because of their proximity to lymphoid tissue, are rudimentary and, therefore, more easily penetrated by macromolecules than epithelial cells elsewhere in the intestine. Therefore, although the intestine is generally designed to resist particle penetration in order to protect against infection, the M cells are able to endocytose small amounts of particulate matter from the gut lumen for presentation to the gut associated lymphoid tissue (GALT). This enables the gut immune system to respond to undesirable antigens present in the gut lumen (Neutra and Kraehenbuhl, *Trends in Cell Biology*, **2**, 134-138 (1992)).

The function and mode of action of M cells have been extensively studied and semi-quantitative estimates of M cell ability to adsorb particles from intestinal loops were first carried out using fluorescent microspheres. From this work it was suggested that these beads might prove useful in studying antigen uptake adsorbed onto polystyrene microparticles (Pappo and Ermak, *Cellular and Experimental Immunology*, **76**, 144 (1989)). It was subsequently found that coating particles with an anti-M-cell IgM antibody causes a three fold increase in bead uptake (Pappo *et al*., *Immunology*, **73**, 277 (1991)). In addition, it was shown that IgA raised against mammary tumour virus increased the binding of the IgA-virus complex to the M cell surface and IgG inhibited the binding. (Weltsin *et al*., *J*. *Cell Biol*., **108**, 1673 (1989). More recent work has shown IgA-coated beads to bind four times more readily and to be taken up twenty times more readily than beads coated with albumin (Porta *et al*., *Exp*. *Physiol*., **77**, 929 (1992)). It has therefore been concluded that IgG, IgA and IgM binding to beads and/or particles can be expected to increase the binding of the beads or particles and their uptake by M cells.

However, although it has been suggested in the past that M cells might be useful as a route for the absorption of macromolecules (O'Hagan, *Advanced Drug Delivery Reviews*, **5**, 265-285 (1990)), this idea has not been developed further and all of the recent work concerning the transporting of beads into M cells has been concentrated on the binding of beads to M cells as a model for antigens which it is normally the function of M cells to take up and process. The idea of using M cells as a possible route by which proteins and peptides could pass from the gut lumen into the body seems not to have been afforded serious consideration, possibly because of anticipated problems in transporting macromolecules out of M cells into the lymphatic system.

Recent work on M cells has concentrated on the coating of beads or particles with immunoglobulins raised against some component of the M cell surface. However, in an attempt to use the M cells to direct proteins or peptides from the gut lumen into lymphoid tissue, the present inventor has adopted an alternative approach.

In a first aspect of the present invention, there is provided a pharmaceutical or veterinary composition for oral administration, the composition comprising a biologically active material, an antibody which is specifically bound to the material and a plurality of polymer beads.

The theory behind the use of such a composition is that an antibody raised against a biologically active peptide or protein will not only bind specifically to that peptide or protein but will also bind non-specifically to the M cell surface so allowing a bead or particle carrying the peptide or protein and the antibody to be carried into and across M cells. The dual action of specific binding to the active peptide or protein and non-specific binding to the M cell surface should preserve the selectivity of the absorption whilst increasing the gut permeability in an immunoglobulin non-selective manner. It should be stressed, however, that the efficacy of the invention is in no way affected by the correctness or otherwise of this theory.

As briefly mentioned above, it might have been expected that even if beads or particles could be transported into M cells, they would remain there and would not pass out of the M cells into the lymphatic system. Surprisingly, however, it now seems that this is not a major problem and that it is possible to gain access to the lymphatic system *via* M cells.

The greatest advantage of the composition of the present invention is, of course, that it makes possible the oral administration of biologically active materials. However, a further advantage is that, from the M cells, the peptide or protein will pass into the lymphatic system rather than into the blood and this may mean that bioavailability is increased because hepatic first pass metabolism will be avoided. In addition, the composition of the present invention is relatively inexpensive and easy to prepare.

The term 'biologically active material' includes pharmaceutically active materials and materials which modify biological function, particularly proteins and peptides and also molecules comprising proteins or peptides, for example, glycoproteins which contain both protein and sugar residues. Other biologically active materials which may be present in the compositions include nucleic acids, for example oligonucleotides such as antisense oligonucleotides which may be useful for interfering with the replication of nucleic acids in virally infected or cancerous cells and for correcting other forms of inappropriate cell proliferation. Polysaccharides such as heparin are also suitable for inclusion in the compositions as are combinations of one or more protien, nucleic acid or polysaccharide.

The material may be useful in human or veterinary medicine and may be used either for the treatment or the prophylaxis of diseases. Alternatively, the materials may be useful for cosmetic or for diagnostic purposes.

When the biologically active material is a protein or peptide, it may be a substance which occurs naturally in the human or animal body, for example, a protein hormone such as insulin, calcitonin or a growth hormone or a protein such as erythropoietin, plasminogen activators and their precursors, for example, t-PA urokinase, prourokinase and streptokinase, an interferon, for example, human interferon-α, or an interleukin such as IL-1, IL-2, IL-3, IL-4 or IL-5 a blood factor such as factor VIII.

Alternatively, the protein or peptide may be intended as a vaccine, in which case it will be antigenic and may be all or part of a Protein derived from a pathogenic organism, for example a bacterial protein or a viral coat protein. In either case, the protein or peptide may be isolated from natural sources, produced by genetically modified organisms or may be wholly or partially synthesised by automated means.

For vaccines, it is a particular advantage that when the composition of the invention is used, the biologically active material is passed directly into the lymphoid tissue since it is quickly available to the immune system which can then raise antibodies specific for the administered peptide or protein and thereby give the body immunity against the organism from which the peptide or protein is derived.

The antibody may be of the IgG, IgA or IgM isotype although IgG is preferred. As mentioned above, it is specific for the biologically active material and may be prepared by conventional means. Methods for raising antibodies are well known to those skilled in the art.

The biologically active material will generally be adsorbed onto the surface of the beads and the antibody is added so as to form a complex between the material and the antibody and to leave part of the antibody free for non-specific binding to the M cells. Perhaps one of the most surprising aspects of the present invention is that it is not necessary to add the active material and the antibody in equal quantities to the surface of the beads. Instead, it is possible to use a considerable excess of the active material and this, of course, allows a greater amount of the material to be endocytosed with the beads than would have been expected.

The ratio of active material to antibody may vary considerably but may be from as high as about 750:1 to 1:1. More generally, the ratio will be from about 500:1 to 10:1 and a typical value is about 100:1.

The beads may be either solid or hollow and may, optionally, be biodegradable. Biodegradable beads can be prepared from homo- or co-polymers of naturally occurring materials and examples of such polymers include poly(D,L-lactide-co-glycolides) in which the proportions of lactide and glycolide can be varied according to the exact properties of which are required. Biologically active materials such as proteins may be incorporated into biodegradable beads, for example in the cavities of hollow beads, and will be released when the beads break down. The beads may be chosen to break down after a predetermined time - for example after the beads have passed through the M cells into the lymphatic system.

The beads are made from a non-immunogenic polymeric material such as polystyrene, latex or other polymers and range from 0.05 µm to 10 µm in diameter. A particularly suitable diameter of bead is from 0.1 to 1.5 µm.

Suitable beads may be obtained from Polysciences, Inc. (400 Valley Road, Warrington, PA18976, USA).

The composition may be formulated as tablets and may contain other excipients, for example fillers, binders or colouring or flavouring agents. However, because the composition takes the form of beads, it is preferred that it is packed into capsules which may be of either the hard shelled or the soft shelled type.

Preferably the composition is enteric coated to enable it to pass through the stomach without being broken down in order that the greatest possible number of beads should reach the Peyer's patch tissue. Suitable enteric coating materials are well known to those skilled in the art.

The dosage of the composition may easily be determined by one skilled in the art and will be similar to the dosages of proteins and peptides which are, at present, administered by other routes.

In a second aspect of the present invention, there is provided a process for the preparation of a pharmaceutical composition according to the first aspect, the process comprising adsorbing a biologically active material onto polymer beads and reacting the material with an immunoglobulin which binds specifically to it.

The steps may be carried out in either order - that is an antibody complex may be prepared and then adsorbed on the surface of the beads or, alternatively, the biologically active material may be adsorbed onto the surface of the beads, following which, the beads are incubated with an immunoglobulin which binds specifically to the peptide or protein. In general, however, it is preferred that the beads be incubated with the biologically active material, washed to remove unbound material and then separately incubated with the antibody.

The amount of biologically active material which is adsorbed onto the surface of the beads varies depending on the concentration of the active material in the solution in which the beads are incubated. It has been found that a suitable concentration of the active material is from about 3 to about 10 mg/ml and preferably from 4 to 6 mg/ml. For proteins such as bGH, bSA and hIgG, it has been found that saturation of the beads is achieved using solutions of concentration greater than about 5 mg/ml.

The amount of biologically active material which is adsorbed also, of course, depends on the size of the beads used. Clearly, the smaller the beads, the larger the surface area per unit weight of bead.

When the above described method of preparing the formulation is used, it may be necessary to incubate the beads a second time in a solution of active material before incubation with the antibody solution in order to increase the amount of active material adsorbed onto the surface of the beads. If this second incubation step is used, it is often useful to use a much higher concentration of active material in the incubation solution and a suitable concentration may be, for example from 30 to 80 mg/ml, typically about 50 mg/ml.

The process may include the additional steps of encapsulating the beads and coating with an enteric coat.

The invention will now be further described with reference to the following examples and to the accompanying drawing.

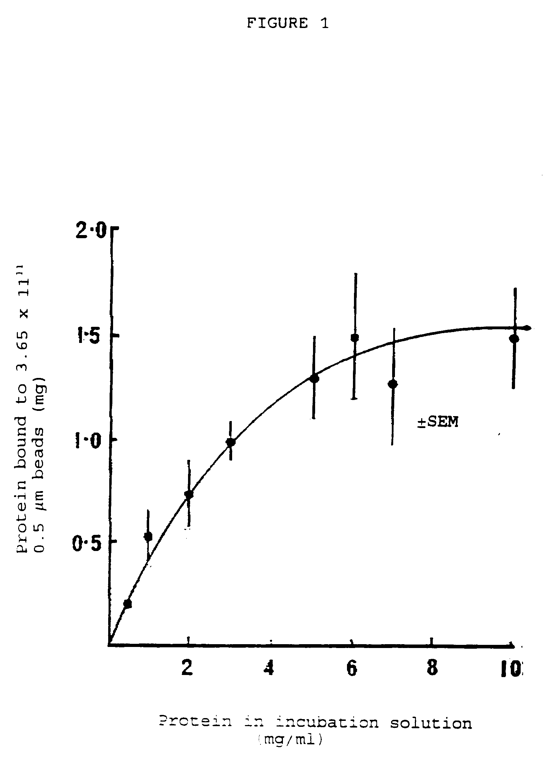

FIGURE 1 is a plot of the amount of protein which binds to a fixed number of beads against the concentration of protein in the incubation solution.

### Example 1

### Preparation of Coated Beads

FLUORESBRITE™ polystyrene plain 0.5 µm microspheres (Polysciences, Inc, No 17152, yellow-green and No 19507, red) were incubated separately in pH 11.0 buffer adjusted to pH 7.4 in the presence of 5 mg/ml bovine serum albumin (bSA), human immunoglobulin G (hIgG) or bovine growth hormone (bGH) for 90 min at 37°C. Protein coated beads were then washed twice with phosphate buffered saline and unbound surface sites blocked by a second incubation in a 50 mg/ml solution of bSA for 30 min at 37°C. These beads were then washed three times in phosphate buffered saline before further use. Binding specific IgG antibody raised against bGH to the bGH coated beads involved a further incubation with this antibody for 3 hr at 37°C. Attachment of the antibody to bGH was verified, in separate experiments, by estimating the amount of I¹²⁵-labelled antibody bound to similar bGH-coated beads.

Suspensions of red or yellow-green protein beads coated with different proteins were then mixed, in equal proportions, to produce a final suspension containing 7.2 x 10¹¹ beads/ml for instillation into mouse intestinal loops containing Peyer's patch tissue.

Experiments to optimise the amount of protein bound to the beads in the first incubation were carried out and a plot of bound protein against protein concentration in the incubation solution was prepared in order to calculate the optimum protein concentration to be used. This plot is shown in Figure 1. Incubation of 0.5 µm beads was carried out using three different proteins, bGH, bSA and hIgG in solutions of concentration varying from 1 to 10 mg/ml. For a standard number of 3.65 x 11¹¹ 0.5 µm beads, it was found that the maximum amount of protein which could be bound varied from about 0.2 to 1.5 mg and that saturation of the beads was achieved using solutions of concentration greater than about 5 mg/ml.

### Example 2

### Absorption of Beads Into M Cells

Eight-to-ten week old Balb/c mice were anaesthetized with 0.4 ml avertin (2.5% w/v) injected intraperitoneally and closed loops of distal ileum formed containing 7.2 x 10¹¹ beads per ml of Krebs-Henseleit buffer, pH 7.4. Peyer's patch tissue removed from these loops 45 min later was then rinsed in buffer and fixed in 4% v/v glutaraldehyde. This tissue was then stained with 5% w/v propidium iodide to aid later identification of M cells using confocal microscopy. Confocal images taken serially at 4 µm intervals through the epithelium were captured under conditions enabling red and yellow-green fluorescent beads to be counted separately. It was also possible, using this method, to count the number of beads binding to the surface of M cells separately from those which had already entered M cells.

Experiments were carried out to study the interaction of microbeads with mouse intestinal M cells. Three different experiments were carried out. In the first experiment, the uptake of free bovine growth hormone (bGH) was compared with the uptake of bovine serum albumen (bSA). In the second experiment, the uptake of bSA was compared with the uptake of human immunoglobulin (hIgG) and in the third experiment, the uptake of free bGH was compared with the uptake of bGH bound to a specific antibody. The results obtained from this experiment are set out in Tables 1 and 2 below.

**Table 1**

| Experiment | Surface Binding | | |
|---|---|---|---|
| 1 | bGH 188 ± 57 (8) | bSA 296 ± 79 (8) | bSA/bGH 1.7 ± 0.1 (8) |
| 2 | bSA 39 ± 15 (8) | hIgG 117 ± 27 (8) | hIgG/bSA 5.4 ± 1.8 (8) |
| 3 | bGH 234 ± 70 (8) | bGH-Ab 436 ± 61 (8) | bGH-Ab/bGH 3.3 ± 0.6 (8) |

**Table 2**

| Experiment | Cellular Uptake | | |
|---|---|---|---|
| 1 | bGH 45 ± 14 (8) | bSA 84 ± 22 (8) | bSA/bGH 2.2 ± 0.4 (8) |
| 2 | bSA 28 ± 10 (8) | hIgG 102 ± 62 (8) | hIgG/bSA 5.7 ± 1.1 (8) |
| 3 | bGH 39 ± 13 (8) | bGH-Ab 214 ± 44 (8) | bGH-Ab/bGH 9.5 ± 2.0 (8) |

All of the values given in Tables 1 and 2 are means ± SEM estimated over 3 x 10⁴ µm² of intestinal follicle surface. The values given in brackets represent the number of follicles analysed.

The ratios given in Tables 1 and 2 were calculated separately for each follicle analysed and so do not correspond exactly to the ratio of the figures in the first and second columns.

The first experiment shows that beads coated with bSA bind to and enter M cells about twice as readily as those coated with bGH after both sets of beads have been presented simultaneously to the same piece of tissue in an intestinal loop. The second experiment shows that, under identical conditions, beads coated with hIgG bind and enter M cells over five times more readily than beads coated with bSA. This type of non selective effect has been seen previously using IgG, IgA and IgM proteins.

The third experiment shows that beads coated with bGH to which a specific antibody is later bound is taken up about ten times more readily than are beads coated with bGH alone even though the increased efficiency of binding is only three-fold. This was a most unexpected result and the mechanism leading to this selective increase in cell uptake remains unknown.

The statistical significance of the results shown in Tables 1 and 2 was assessed and the results of this assessment are shown in Table 3.

**Table 3**

| P values (two-tailed paired t-test) | | | |
|---|---|---|---|
| **Testing** | **Surface binding** | **Cellular uptake** | **selectivity** |
| 1. bSA > bGH | 0.003 | 0.029 | 0.178 (NS) |
| 2. hIgG > bSA | 0.007 | 0.000 | 0.078 (NS) |
| 3. bGH-Ab > bGH | 0.001 | 0.000 | 0.009 |

Table 3 indicates the statistical significance of protein selective effects on microbead binding and uptake by mouse intestinal M cells.

The following results are indicated:
Surface binding: bGH < bSA < hIgG; bGH <bGH-Ab
Cellular uptake: bGH< bSA < hIgG; bGH < < bGH-Ab
Selectivity: uptake > binding: bGH = bSA = hIgG; bGH < < bGH-Ab. (NS) = not statistically significant.

The experimental protocol employed allows one to assess the significance of differences reported in Table 1 using a paired t-test. This in turn removes any error that could occur through animal or tissue variation. All of the protein dependent differences in bead binding and uptake were statistically significant and the additional increase in bead uptake conferred by binding a specific antibody to bGH was also found to be statistically significant (P = 0.009).

From the results of the above experiments, it can be seen that the amount of protein entering the M cells is significantly increased when antibody complexed to the protein is adsorbed onto the beads.

### Example 3

### Movement of Beads Through M Cells

The experiment described in Example 2 was repeated using rats rather than mice. In this experiment, 1 ml of phosphate buffered saline (PBS) containing 7.2 x 10¹¹ beads per ml was instilled into the proximal gut lumens of 11 rats. The gut contents were then allowed to flow distally under as nearly physiological conditions as possible.

In five of the rats, Peyer's patch tissue removed from the gut lumen after 90 minutes was rinsed and stained before taking confocal images at 4 µm intervals through the epithelium in order to determine the number of beads binding to M cells and the number of beads which had already entered the M cells.

In the remaining six rats, the mesenteric lymph ducts were cannulated before instilling the bead suspension into the proximal gut lumen and samples of lymph were taken at 5 minute intervals for 90 minutes. The samples were then scanned using a fluorescent sorter (FACS analysis) to determine the presence of red and green beads.

In this experiment, the uptake and throughput of free bovine growth hormone (bGH) was compared with similar results for bGH bound to a specific antibody (bGH-Ab). The results are presented in Table 4.

**Table 4**

| **Ratio GH-Ab:GH** | **Method** | **Result** |
|---|---|---|
| Cell Surface Binding | Confocal | 3.1 ± 0.6 (5) |
| Cellular Uptake | Confocal | 5.0 ± 0.3 (5) |
| Entering Lymph | FACS Analysis | 2.9 ± 0.4 (6) |

The results presented in Table 4 confirm the results of Example 2 by showing once again that beads coated with bGH to which a specific antibody is later bound will bind to M cells more efficiently than beads coated with bGH alone. Furthermore, the uptake of the bGH-Ab coated beads is even more significant with, in this case, about five times as many bGH-Ab coated beads entering M cells as bGH coated beads. The results also show, however, that about three times as many beads coated with bGH-Ab reached the lymphatic system as beads coated with bGH alone. This suggests that the bioavailability of a pharmaceutically active substance would be significantly improved by its inclusion in a composition according to the present invention because the active substance is able to pass preferentially into the lymphatic system thus avoiding first pass metabolism which occurs if active substances are absorbed into the hepatic portal vein and are passed directly to the liver.

## Claims

1. A pharmaceutical or veterinary composition for oral administration, the composition comprising a biologically active material, an antibody which is specifically bound to the biologically active material and a plurality of polymer beads.

2. A pharmaceutical composition as claimed in claim 1, wherein the biologically active material is a peptide or protein.

3. A composition as claimed in claim 2, wherein the protein or peptide is one which occurs naturally in the human or animal body for example a protein hormone such as insulin, calcitonin or a growth hormone or a protein such as erythropoietin, plasminogen activators and their precursors, for example, t-PA urokinase, pro-urokinase and streptokinase, an interferon, for example, human interferon-α, or an interleukin such as IL-1, IL-2, IL-3, IL-4 or IL-5 a blood factor such as factor VIII.

4. A composition as claimed in claim 2, wherein the protein or peptide is for use as a vaccine and comprises all or part of a protein derived from a pathogenic organism.

5. A composition as claimed in any one of claims 1 to 4 wherein the antibody is of the IgG, IgA, or IgM isotype.

6. A composition as claimed in any one of claims 1 to 5 wherein the beads are made from polystyrene.

7. A composition as claimed in any one of claims 1 to 5 wherein the beads range in size from 0.5 µm to 10 µm.

8. A composition as claimed in any one of claims 1 to 7 wherein the active material and the antibody form a complex which is adsorbed onto the surface of the beads.

9. A composition as claimed in any one of claims 1 to 8, wherein the ratio of active material to antibody is from 750:1 to 1:1.

10. A composition as claimed in any one of claims 1 to 9 wherein the beads are biodegradable.

11. A composition as claimed in claim 10 wherein the biodegradable beads contain additional protein or peptide.

12. A composition as claimed in any one of claims 1 to 11 which has an enteric coat.

13. A process for the preparation of a pharmaceutical composition comprising a biologically active material, an antibody which is bound specifically to the active material and a plurality of polymer beads; the process comprising adsorbing the biologically active material onto the polymer beads and then reacting the active material with the antibody.

14. A process for the preparation of a pharmaceutical composition comprising a biologically active material, an antibody which is bound specifically to the active material and a plurality of polymer beads; the process comprising binding the antibody to the biologically active material and then adsorbing the bound antibody onto the beads.

15. A process as claimed in claim 13 or claim 14, further comprising enterically coating the composition.

## Patentansprüche

1. Pharmazeutische oder veterinärmedizinische Zusammensetzung zur oralen Verabreichung, wobei die Zusammensetzung ein biologisch aktives Material, einen an das biologisch aktive Material spezifisch gebundenen Antikörper und eine Mehrzahl von Polymerkörnern enthält.

2. Pharmazeutische Zusammensetzung nach Anspruch 1, bei welcher das biologisch aktive Material ein Peptid oder ein Protein ist.

3. Zusammensetzung nach Anspruch 2, bei welcher das Protein oder Peptid ein solches ist, das im menschlichen oder tierischen Körper natürlich vorkommt, z. B. ein Proteinhormon wie Insulin, Calcitonin oder ein Wachstumshormon, oder ein Protein wie Erythropoietin, Plasminogenaktivatoren und ihre Vorläufer, z.B. t-PA Urokinase, pro-Urokinase und Streptokinase, ein Interferon, z.B. menschliches α-Interferon, oder ein Interleukin wie IL-1, IL-2, IL-3, IL-4 oder IL-5 bzw. ein Blutfaktor wie Faktor VIII.

4. Zusammensetzung nach Anspruch 2, bei welcher das Protein oder Peptid für den Gebrauch als Vaccine geeignet ist und das gesamte oder Teile eines Protein enthält, das von einem pathogenen Organismus stammt.

5. Zusammensetzung nach einem der Ansprüche 1 bis 4, bei welcher der Antikörper ein IgG, IgA oder IgM Isotyp ist.

6. Zusammensetzung nach einem der Ansprüche 1 bis 5, bei welcher die Körner aus Polystyrol bestehen.

7. Zusammensetzung nach einem der Ansprüche 1 bis 5, bei welcher die Körner in der Größenordnung von 0,5 µm bis 10 µm sind.

8. Zusammenfassung nach einem der Ansprüche 1 bis 7, bei welcher das aktive Material und der Antikörper einen Komplex bilden, der an der Oberfläche der Körner adsorbiert ist.

9. Zusammensetzung nach einem der Ansprüche 1 bis 8, bei welcher das Verhältnis von aktivem Material zu Antikörper von 750:1 bis 1:1 beträgt.

10. Zusammensetzung nach einem der Ansprüche 1 bis 10, bei welcher die Körner biologisch abbaubar sind.

11. Zusammensetzung nach Anspruch 10, bei welcher die biologisch abbaubaren Körner zusätzlich ein Protein oder Peptid enthalten.

12. Zusammensetzung nach einem der Ansprüche 1 bis 11, welche einen enterischen Überzug besitzt.

13. Verfahren zur Herstellung einer pharmazeutischen Zusammensetzung, die ein aktives Material, einen spezifisch an das aktive Material gebundenen Antikörper und eine Mehrzahl von Polymerkörnern aufweist; wobei das Verfahren das Adsorbieren des biologisch aktiven Materials an die Polymerkörner und das Reagieren des aktiven Materials mit dem Antikörper umfaßt.

14. Verfahren zur Herstellung einer phamazeutischen Zusammensetzung, die ein aktives Material, einen spezifisch an das aktive Material gebundenen Antikörper und eine Mehrzahl von Polymerkörnern aufweist; wobei das Verfahren das Binden des Antikörpers an das biologisch aktive Material und das nachfolgende Adsorbieren des gebundenen Antikörpers an die Körner umfaßt.

15. Verfahren nach Anspruch 13 oder 14, welches weiters das enterische Überziehen der Zusammensetzung umfaßt.

## Revendications

1. Composition pharmaceutique ou vétérinaire pour l'administration orale, la composition comprenant une substance biologiquement active, un anticorps qui est lié de manière spécifique à la substance biologiquement active et une pluralité de billes de polymère.

2. Composition pharmaceutique selon la revendication 1, dans laquelle la substance biologiquement active est un peptide ou une protéine.

3. Composition selon la revendication 2, dans laquelle la protéine ou le peptide est une substance qui apparait naturellement dans le corps humain ou animal par exemple une hormone protéinique comme l'insuline, la calcitonine ou une hormone de croissance ou une protéine comme l'érythropoïétine, les activateurs du plasminogène et leurs précurseurs, par exemple, la t-PA urokinase, la pro-urokinase et la streptokinase, un interféron, par exemple, l'interféron humain α, ou une interleukine comme IL-1, IL-2, IL-3, IL-4 ou IL-5, un facteur sanguin comme le facteur VIII.

4. Composition selon la revendication 2, dans laquelle la protéine ou le peptide est destiné à l'utilisation comme vaccin et comprend la totalité ou une partie d'une protéine dérivée d'un organisme pathogène.

5. Composition selon l'une quelconque des revendications 1 à 4, dans laquelle l'anticorps est de l'isotype IgG, IgA ou IgM.

6. Composition selon l'une quelconque des revendications 1 à 5, dans laquelle les billes sont des billes de polystyrène.

7. Composition selon l'une quelconque des revendications 1 à 5, dans laquelle la dimension des billes varie de 0,5 µm à 10 µm.

8. Composition selon l'une quelconque des revendications 1 à 7, dans laquelle la substance active et l'anticorps forment un complexe qui est adsorbé sur la surface des billes.

9. Composition selon l'une quelconque des revendications 1 à 8, dans laquelle le rapport de la substance active à l'anticorps est de 750:1 à 1:1.

10. Composition selon l'une quelconque des revendications 1 à 9, dans laquelle les billes sont biodégradables.

11. Composition selon la revendication 10, dans laquelle les billes biodégradables contiennent une protéine ou un peptide supplémentaire.

12. Composition selon l'une quelconque des revendications 1 à 11, qui possède un enrobage entérique.

13. Procédé de préparation d'une composition pharmaceutique comprenant une substance biologiquement active, un anticorps qui est lié de manière spécifique à la substance active et une pluralité de billes de polymère; le procédé comprenant l'adsorption de la substance biologiquement active sur les billes de polymère et ensuite la réaction de la substance active avec l'anticorps.

14. Procédé de préparation d'une composition pharmaceutique comprenant une substance biologiquement active, un anticorps qui est lié de manière spécifique à la substance active et une pluralité de billes de polymère; le procédé comprenant la liaison de l'anticorps à la substance bioloqiquement active et ensuite l'adsorption de l'anticorps lié sur les billes.

15. Procédé selon la revendication 13 ou la revendication 14, comprenant en outre l'enrobage entérique de la composition.
